# EUROPEAN PATENT SPECIFICATION

(11) **EP 2 288 413 B1**
(45) Date of publication and mention of the grant of the patent: **14.03.2012**
(21) Application number: 08802825.3
(22) Date of filing: 08.10.2008
(51) Int. Cl.: A61N 5/10, A61N 1/40

(54) **THERAPEUTIC DEVICE COMBINING RADIATION THERAPY AND THERMOTHERAPY**
THERAPEUTISCHE VORRICHTUNG FÜR KOMBINIERTE STRAHLENTHERAPIE UND THERMOTHERAPIE
DISPOSITIF THERAPEUTIQUE COMBINANT RADIOTHERAPIE ET THERMOTHERAPIE

(43) Date of publication of application: 02.03.2011
(73) Proprietor: Helmholtz Zentrum München Deutsches Forschungszentrum für Gesundheit und Umwelt (GmbH), 85764 Neuherberg (DE)
(72) Inventor: ISSELS, Rolf, 80796 München (DE)
(74) Representative: Beier, Ralph
(86) International application number: PCT/EP2008/008488
(87) International publication number: WO 2010/040364

(56) References cited:
- EP-A- 0 719 571
- WO-A-00/59576
- WO-A-01/60236
- WO-A-02/45790
- WO-A-2007/067830
- GB-A- 1 045 546
- JP-A- 9 099 097
- MONTES H ET AL: "A SYSTEM FOR THE SIMULTANEOUS DELIVERY OF INTRAOPERATIVE RADIATION AND ULTRASOUND HYPERTHERMIA" INTERNATIONAL JOURNAL OF HYPERTHERMIA, BASINGSTOKE, GB, vol. 11, no. 1, 1 January 1995 (1995-01-01), pages 109-119, XP008022212 ISSN: 0265-6736

## Description

### Field of the invention

The invention relates to a therapeutic device for treatment of a patient, particularly for cancer treatment.

### Background of the invention

One of the conventional therapies against cancer is radiation therapy which uses ionizing radiation to kill cancer cells and shrink tumors. The radiation can be administered externally by external beam radiotherapy (EBRT) or internally by the so-called brachytherapy.

A further development of the external beam radiotherapy is the so-called tomotherapy where the radiation is delivered to the patient helically by a radiation source which is rotating around the patient. An advantage of the tomotherapy is the ability to precisely deliver radiation to a cancerous tumor while sparing the normal healthy tissue around it.

Another treatment against cancer is the so-called thermotherapy where a localized or regional hyperthermia is induced in the patient in the area of the cancerous tumor.

One positive effect of thermotherapy is the improvement of the blood supply and therefore the oxygenation in the cancerous tumor so that the tumor cells are more responsive to a following radiation therapy or chemotherapy.

Further, the thermotherapy impairs the ability of the cancerous tumor cells to repair damages caused by a preceding radiation therapy.

It is further known to combine radiation therapy and thermotherapy to improve the therapy results. However, it is disputed whether the thermotherapy should be administered to the cancer patient before or after the radiation therapy.

In some clinics, the cancer patients are first subjected to a thermotherapy. Then, the cancer patients are brought to another room where the radiation therapy is administered to the patients in a specialized radiation therapy apparatus, e.g. a tomotherapy apparatus.

In other clinics, the cancer patients are first subjected to the radiation therapy, e.g. in a tomotherapy apparatus. Then, the cancer patients are brought to another room where the thermotherapy is administered to the cancer patients in order to impair the ability of the cancerous tumor cells to repair the damages caused by the preceding radiation therapy.

However, the afore-mentioned conventional cancer therapies are not entirely satisfactory.

Further, reference is made to WO 2007/067830 A, WO 00/59576 A, WO 01/60236 A, EP 0 719 571 A, WO 02/45790 A, JP 09 099097 A, Montes: "A system for the simultaneous delivery of intraoperative radiation and ultrasound hyperthermia", International Journal of Hyperthermia, vol. 11, no. 1 (1995) and GB-A-1 045 546.

However, none of these prior art references discloses the novel idea to administer an image-guided thermotherapy to avoid temperature hotspots and/or to achieve a desired special temperature distribution within the body interior.

### Summary of the invention

Therefore, it is a general object of the invention to provide an improved therapeutic device for treatment of cancer patients.

This object is achieved by a novel therapeutic device as defined in claim 1.

The invention comprises the general technical teaching that the radiation therapy and the thermotherapy should be applied to the cancerous cells spatially and temporally simultaneously. Therefore, the invention is distinguishable from the afore mentioned conventional combination therapies where the thermotherapy is administered to the cancer patients either before the radiation therapy or after the radiation therapy.

Therefore, the therapeutic device according to the invention comprises both a radiation therapy apparatus and an integrated thermotherapeutic heating device. The radiation therapy apparatus applies an ionizing radiation to the patient, while the thermotherapeutic heating device induces a regional hyperthermia in the patient.

The term hyperthermia defines a procedure in which the body cells in the area of treatment are heated to a temperature of 40°C-46°C. It should further be noted that the thermotherapeutic heating device induces a regional hyperthermia only. Therefore, the hyperthermia is spatially limited to the area of treatment while the body cells outside the area of treatment remain at a lower temperature near the normal body temperature.

Further, the radiation therapy apparatus and the thermotherapeutic heating device are preferably adapted to operate simultaneously, so that a regional area of treatment within the patient can be subjected to a radiation therapy and a regional hyperthermia simultaneously.

Further, the radiation therapy apparatus preferably administers an external beam radiotherapy (EBRT) to the patient. However, the invention is not restricted to an external beam radiotherapy. Alternatively, the ionizing radiation can be delivered to the patient in other ways, e.g. by a probe which is inserted into the patient.

Further, the radiotherapy apparatus preferably administers an intensity modulated radiation therapy (IMRT) to the patient, which is well-known in the state of the art. Therefore, the radio therapy apparatus generates a dose distribution which is well adapted to the target volume (e.g. a tumor) while sparing healthy tissues according to radiobiological considerations.

It should further be noted that the radiation therapy apparatus administers an image-guided radiation therapy (IGRT) to the patient by the use of images of the body interior of the patient, wherein the images are generated by a body scanner, e.g. a computer tomograph (CT). Preferably, X-ray beam are utilized to generate a so-called MV-CT (megavolt computer tomograph) or cone-beam CT before starting the radiotherapy. This MV-CT is preferably matched with the previously generated planning CT to correct the position of the patient for the actual radiotherapy, which is called interfractional image guidance.

Further, the radiation therapy apparatus is preferably a tomotherapy apparatus which is adapted for an image-guided and/or intensity modulated delivery of beams of the ionizing radiation by rotating around the patient slice-by-slice thereby employing all directions.

Further, the tomotherapy apparatus preferably comprises a multi-leaf collimator for matching the radiation field to the shape of the tumor by modulating the dose distribution according to the shape of the tumor. Suitable tomotherapy apparatuses are commercially available from the company Tomotherapy Inc. (USA).

The thermotherapeutic heating device induces the regional hyperthermia within the patient preferably by depositing ultrasound, electromagnetic waves, particularly radio frequency waves or microwaves, into the patient. The afore mentioned radiation is preferably radiated by an antenna arrangement which is preferably annular or ring-shaped and surrounds the patient. The antenna arrangement preferably uses the interference principle and surrounds the patient. Suitable thermotherapeutic heating devices are commercially available from the company BSD Medical Corporation (USA).

Alternatively, the cancerous cells can be heated by a probe which can be inserted into the patient to an area of treatment, wherein the probe locally or regionally heats the patient in the area of treatment so that a regional hyperthermia is induced in the cells within the area of treatment.

The device for image-guidance of radiotherapy, e.g. megavolt computer tomograph (MV-CT) is also useful to control the heat treatment distribution. Basically, the MV-CT generates a three-dimensional density distribution of the patient characterized by the so-called Hounsfield units (HU) which has a direct relationship to the absorption coefficient µ of the tissue: HU(x, y)=1000 · (µ(x, y)/µ₀-1), wherein the reference tissue µ₀ is water. The density HU is dependent on temperature, i.e. 0.45HU/°C for muscle tissue. Therefore, a series of MV-CTs, e.g. before the heat treatment, at the end of the heat-up phase (e.g. after 20 minutes) and later during the plateau provides valuable information about the temperature distribution in addition.

Further, the therapeutic device according to the invention comprises at least one control unit controlling the operation of the radiation therapy apparatus and the operation of the thermotherapeutic heating device according to a predetermined program which is executed in the control unit. The predetermined program corresponds to a treatment plan which is preferably generated in a planning system.

For example, the predetermined program can define the location and the shape of the area of treatment of the thermotherapeutic heating device and/or the radiation therapy apparatus. This allows a matching of the area of treatment to the location and shape of a cancerous tumor which is to be treated.

Firstly, in the planning CT dataset the target volume (tumor and risk areas) and organs of risk are specified. The optimal dose distribution using the multi-leaf collimator (MLC) for intensity-modulation is calculated in the radiotherapy planning system.

Secondly, in a hyperthermia planning system the power deposition pattern and then the temperature distribution is calculated for any given phase and amplitude set of the antennas. Then, the particular phases and amplitudes are predicted providing the best solution for a certain tumor topography.

As a consequence, the predetermined program can define a sequence of operation of the radiation therapy apparatus and/or the thermotherapeutic heating device. In a preferred embodiment of the invention, the predetermined program defines three successive phases of treatment characterized by different operational characteristics of the radiation therapy apparatus and the thermotherapeutic heating device.

In the first phase of the treatment it is preferred that a MV-CT of the patient is used to position the patient and an applicator correctly in the geometry. Then, the thermotherapeutic heating device is switched on and the radiation therapy apparatus is switched off. Therefore, the thermotherapy administered to the cancerous cells in the first phase of the treatment with suitable antenna parameters results in an improvement of the blood supply and the oxygenation of the cancerous cells so that the cancerous cells better respond to the following radiation therapy.

In the second phase of the treatment, it is preferred that both the thermotherapeutic heating device and the radiation therapy apparatus are switched on, so that the cancerous cells are subjected to the ionizing radiation and the hyperthermia simultaneously. It has been found that such a simultaneous administration of the radiation therapy and the thermotherapy results in a particularly high synergism of the radiation and temperature effect in the cells.

In the third phase of treatment, the thermotherapeutic heating device is switched on and the radiation therapy apparatus is switched off. The thermotherapy administered to the patient during the third phase of the treatment advantageously impairs the ability of the cancerous cells to repair damages caused by the preceding radiation therapy.

The afore mentioned three phases of treatment preferably each have a duration in a range between 5 minutes and 30 minutes and more preferably in a range between 15 minutes and 25 minutes. Further, the total accumulated duration of the afore mentioned three phases is preferably in a range between 30 minutes and 90 minutes.

Further, the above mentioned predetermined program can define the dose distribution and/or intensity of the radiation therapy and/or the control of the thermotherapy, wherein the intensity can vary according to a specific temporal profile during the treatment time.

It should further be noted that the control unit(s) preferably controls the position of the regional areas of treatment of the radiotherapy apparatus and the thermotherapeutic heating device in such a way that the regional area of treatment of the radiotherapy apparatus spatially overlaps with the regional area of treatment of the thermotherapeutic heating device. Therefore, the cells in the overlapping area (e.g. the tumor infiltrated tissue) are subjected both to a radiation therapy and a thermotherapy simultaneously.

Further, the therapeutic device according to the invention comprises an integrated body scanner generating images of the body interior of the patient, which is important for the afore mentioned image-guided radiation therapy (IGRT). The body scanner is preferably a computer tomograph (CT) although other types of conventional body scanners can be used, e.g. a magnet resonance tomograph (MRT), a positron emission tomograph (PET), an X-ray apparatus, an ultrasonograph or an ultrasound tomograph. Further, the body scanner can be a combination of the afore mentioned imaging devices
wherein the images of the different imaging devices are fused. For example, an image fusion is possible of the images generated by a computer tomograph (CT) on the one hand and the corresponding images of a magnet resonance tomograph (MRT) on the other hand.

Further, the integrated MV-CT can be performed with a slice thickness of 2-15mm. The data acquisition for one slice (half rotation) needs 10s. Therefore, a volume of 10-30cm length can be scanned in 1-3 minutes for a large slice thickness of 15mm (and for better spatial resolution with larger acquisition times accordingly). While the initial MV-CT is employed for image-guidance of radiotherapy (IGRT, interfractional online correction) a temporal sequence of MV-CT can be used to monitor and control the temperature distribution. Note that the tomotherapy must be interrupted for the MV-CT, but a well-defined pause of irradiation for some minutes is possible. In any CT (kVCT or MV-CT) there is a competition between spatial and temperature resolution. Clearly, the standard deviation of HU (and therefore the contrast resolution) is much better for kV-CT (±5HU for a 2mm pixel size at 140kV) than for MV-CT (±15HU for a 1-2cm pixel size at 3MV).

For any object of the size of one pixel, the low contrast resolution is by far not sufficient to provide a reasonable temperature resolution (derived from 0.45HU/°C). However, spatial integration of pixels over several slices and in the slice, i.e. in all three coordinates, can reduce the standard deviation of the contrast resolution considerably. We find the following potentials from statistical considerations:
a) For voxel sizes of 3-5cm, we achieve a temperature resolution of ±6°C, i.e. we are able to detect areas of 3-5cm extension with >50°C (hot spot detection).
b) For voxel sizes of 5-10cm, we achieve a temperature resolution of better ±3°C, i.e. we are able to verify an effective mean temperature of 43°C in a tumor region and to trace a high (probably too high) temperature level around 43°C in normal tissue such as muscles and fat. The temperature analysis is performed in this way by postprocessing of the MV-CT dataset which is transferred in a standard DICOM (Digital Images and Communications in Medicine) format into the hyperthermia planning program.

The invention and its particular features and advantages will become more apparent from the following detailed description considered with reference to the accompanying drawings.

### Brief description of the drawings

- Figure 1: is a schematical perspective view of a therapeutic device according to the invention combining a tomotherapy apparatus and a thermotherapeutic heating device,
- Figure 2: is a schematic cross section of the therapeutic device of Figure 1,
- Figure 3: is a schematic block diagram of the therapeutic device shown in figure 1,
- Figure 4a, 4B: show a flow chart illustrating the mode of operation of the therapeutic device shown in figures 1 to 3.

### Detailed description of the invention

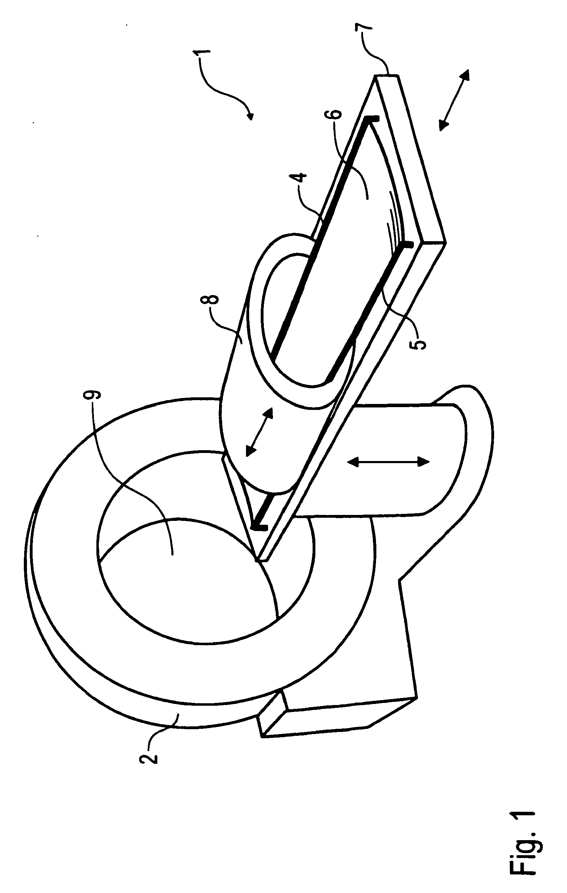

Figures 1-3 illustrate a novel therapeutic device 1 combining radiation therapy and thermotherapy for the treatment of cancer.

Firstly, the therapeutic device 1 comprises a tomotherapy apparatus 2 which administers a tomotherapy to a tumor. The tomotherapy apparatus 2 can be based on a conventional tomotherapy apparatus which is commercially available from the company Tomotherapy Inc. (USA).

The tomotherapy apparatus 2 comprises a treatment table 3 with two lateral metal rods 4, 5 on the top side of the treatment table 3, wherein a mat 6 is spanned between the rods 4, 5 so that a patient 7 can rest on the mat 6 during treatment.

Further, a thermotherapeutic heating device 8 is slidably mounted on the treatment table 3. Before the beginning of the treatment, the thermotherapeutic heating device 8 is moved to the foot end of the treatment table 3 facing the tomotherapy apparatus 2, so that the patient 7 can easily lie down on the mat 6 of the treatment table 3. Then, the thermotherapeutic heating device 8 is moved back to the treatment position in which it surrounds the patient 7 lying on the mat 6.

After the afore-mentioned positioning of the patient 7, the treatment table 3 is moved into an aperture 9 of the tomotherapy apparatus 2 so that the thermotherapeutic heating device 8 is coaxially aligned and centrally located in the aperture 9 of the tomotherapy apparatus 2. In this embodiment, the aperture 9 has a diameter of d=85cm so that the entire treatment table 3 with the patient 7 and the thermotherapeutic heating device 8 can be moved into the aperture 9 of the tomotherapy apparatus 2.

The integrated thermotherapeutic heating device 8 administers a thermotherapy to the cancer patient 7, so that a regional hyperthermia is induced in the patient 7 in the area of treatment. The thermotherapeutic heating device 8 can be based on a conventional thermotherapeutic heating device which is commercially available from the company BSD Medical Corporation (USA).

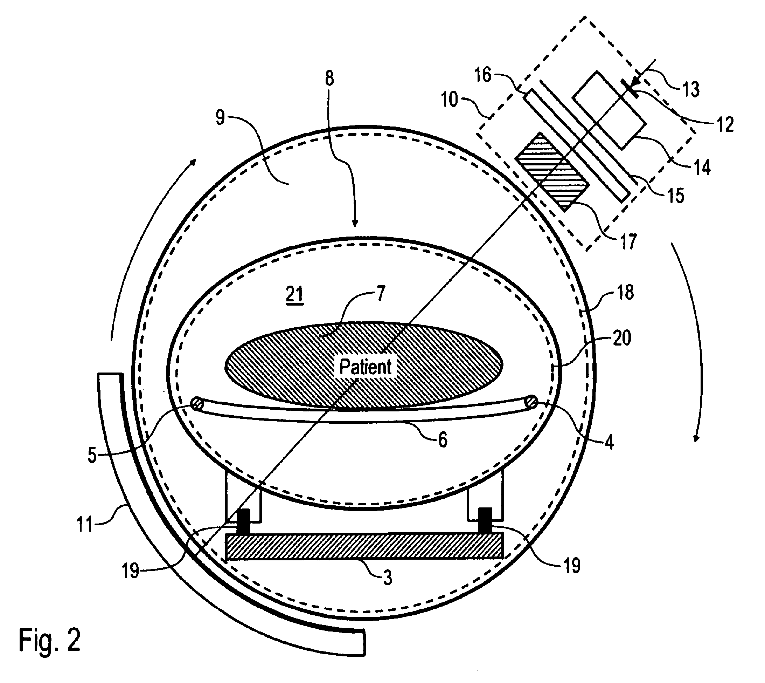

Reference is now made to the cross section shown in Figure 2 showing further details of the thermotherapeutic heating device 8 and the tomotherapy apparatus 2.

The tomotherapy apparatus 2 comprises a radiation unit 10 and a radiation detector 11 which are arranged opposite to each other and rotating around the patient 7. The radiation unit 10 comprises a high-density metallic target 12 (e.g. tungsten) which is hit by an electron beam 13 of 6MeV generated by a linear accelerator (LINAC: Linear accelerator) so that a 6MeV X-ray beam is generated. Further, the radiation unit 10 comprises a primary collimator 14, a flattening filter 15, an ion chamber 16 and a multi-leaf collimator 17 (MLC: Multi-leaf collimator). The primary collimator 14 collimates the X-ray beam in longitudinal direction and the flattening filter 15 homogenizes the X-ray beam. Further, the multi-leaf collimator 17 modulates the dose distribution in the x-z-plane.

Moreover, the inner circumferential wall of the aperture 9 of the tomotherapy apparatus 2 is covered by an electric shielding 18, e.g. a thin copper foil or mesh, which is shielding the electronic components of the tomotherapy apparatus 2.

Moreover, it can be seen that the thermotherapeutic heating device 8 is supported on rolls 19, so that the thermotherapeutic heating device 8 can be rolled along the treatment table 3 to facilitate the positioning of the patient 7 on the mat 6.

Further, it can be seen that the thermotherapeutic heating device 8 comprises an antenna arrangement 20 which is fed by high-frequency cables (not shown). Further, water tubes (not shown) discharge into the interior of the thermotherapeutic heating device 8 to fill the interior with a water bolus 21.

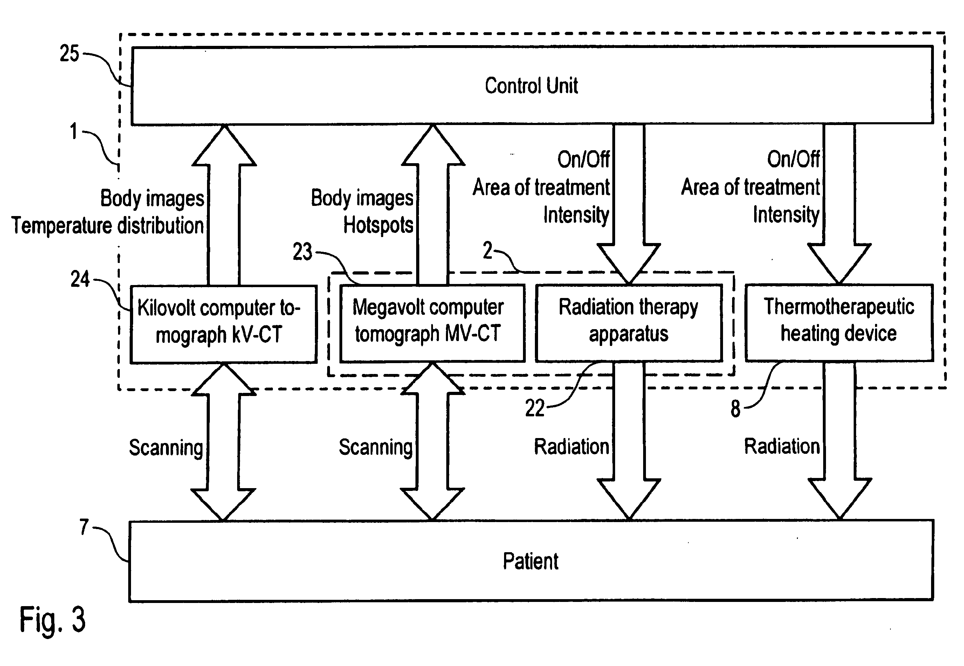

Reference is now made to figure 3 showing a schematic block diagram of the therapeutic device 1 for cancer treatment of the patient 7.

The block diagram shows that the tomotherapy apparatus 2 essentially consists of a radiation therapy apparatus 22 and a megavolt computer tomograph 23 (MV-CT) which generates sectional images of the patient 7 slice-by-slice.

The megavolt computer tomograph 23 is suitable to detect thermal hot spots in the patient 7 which should be avoided during the therapy.

Further, the therapeutic device 1 comprises a kilovolt computer tomograph 24 (kV-CT) which also generates sectional images of the patient 7 slice-by-slice. However, the kilovolt computer tomograph 24 has a much higher spatial resolution than the megavolt computer tomograph 23 so that the temperature distribution in the patient 7 can be determined with high precision.

Further, the therapeutic device 1 comprises a control unit 25 which controls the operation of the radiation therapy apparatus 22 and the thermotherapeutic heating device 8. For example, the control unit 25 either activates or inactivates the radiation therapy apparatus 22 and the thermotherapeutic heating device 8, respectively. Further, the control unit 25 defines the position and shape of the area of treatment of the radiation therapy apparatus 22 and the thermotherapeutic heating device 8 so that the areas of treatment can be matched to the position and shape of the tumor. Moreover, the control unit 25 can modulate the intensity of the radiation which is applied by the radiation therapy apparatus 22 and the thermotherapeutic heating device 8.

The control unit 25 performs an image-guided control of the radiation therapy apparatus 22 and the thermotherapeutic heating device 8, wherein the images of the patient 7 are provided by the kilovolt computer tomograph 24 and the megavolt computer tomograph 23.

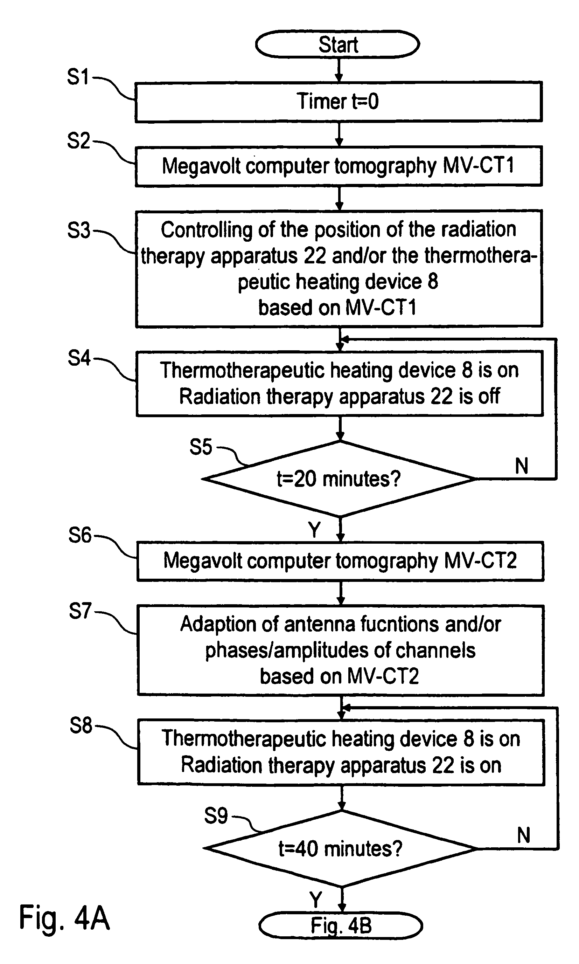

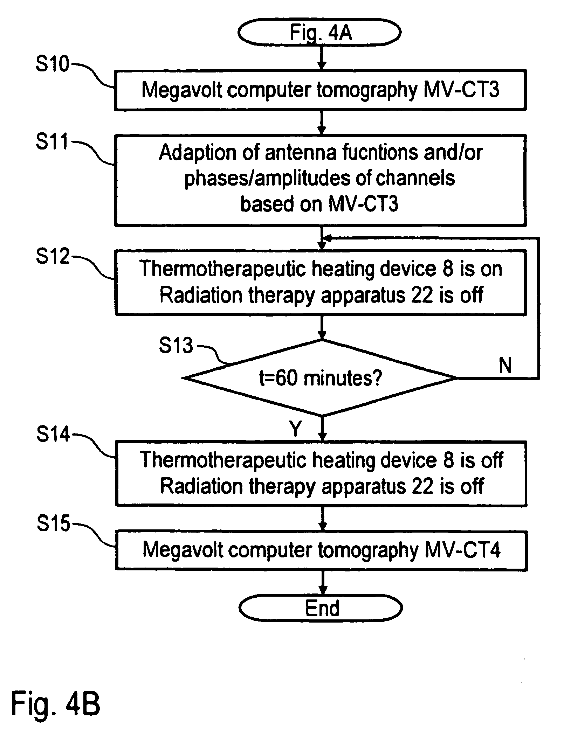

In the following, reference is made to the flowchart shown in figures 4A and 4B.

Before the beginning of a therapy, the patient 7 is placed on the afore-mentioned treatment table 3, so that the thermotherapeutic heating device 8 surrounds the body of the patient 7.

Then, the treatment table 3 along with the patient 7 and the thermotherapeutic heating device 8 is moved into the aperture 9 of the tomotherapy apparatus 2 until the thermotherapeutic heating device 8 is positioned within the aperture 9 of the tomotherapy apparatus 2.

After these preparatory operations, the following treatment is started, wherein the operation of the tomotherapy apparatus 2 and the thermotherapeutic heating device 8 is controlled by a predetermined program which is executed in the control unit 25.

In a first step S1 before the beginning of the treatment, a timer t is resetted.

Then, a first megavolt computer tomography MV-CT1 is performed in a step S2.

The images generated by the first megavolt computer tomography MV-CT1 are then utilized in a step S3 to control and adjust the position of the patient 7 and the thermotherapeutic heating device 8 relative to each other and with regard to the tomotherapy apparatus 2.

In a next step S4, the control unit 25 activates the thermotherapeutic heating device 8 while the radiation therapy apparatus 22 keeps inactive.

In another step S5, the control unit 25 continuously checks whether a first phase of treatment of t=20 minutes already expired.

If the first phase of t=20 minutes has not yet expired, the control unit 25 continues with the step S4 in which the thermotherapeutic heating device 8 is switched on while the radiation therapy apparatus 22 is switched off.

Otherwise, the operation continues with a step S6 in which a second megavolt computer tomography MV-CT2 is conducted. The images generated by the second megavolt computer tomography MV-CT2 are then used in a step S7 to adapt the antenna functions and phases/amplitudes of channels of the therapeutic device 1.

Then, the radiation therapy apparatus 22 is switched on in step S8, while the thermotherapeutic heating device 8 keeps activated. Therefore, the therapeutic heating device 1 administers both a thermotherapy and a radiation therapy to the patient 7.

In a next step S9, the control unit 25 continuously checks whether the second phase of treatment already expired after t=40 minutes.

If so, the control unit 25 continues with step S10 in which a third megavolt computer tomography MV-CT3 is conducted.

The results of the third megavolt computer tomography MV-CT3 are then used in step S11 to adapt the antenna functions and the phases/amplitudes of the channels.

Then, in step S12 the radiation therapy apparatus 22 is switched off while the thermotherapeutic heating device 8 keeps activated.

Further, the control unit 25 continuously checks in step S13 whether the third phase of the treatment already expired after t=60 minutes.

If so, the control unit 25 continues with a step S14 in which both the radiation therapy apparatus 22 and the thermotherapeutic heating device 8 are switched off.

In a final step S15 a fourth megavolt computer tomography MV-CT4 is conducted.

In the following, the operation of the therapeutic device 1 is described in other words to further clarify the invention.

The multimodal cancer treatment performs at first an image-guided and intensity modulated radiotherapy with optimal concentration of the dose in a target (here a locally advanced tumor disease) and well defined sparing of normal tissues and particular critical organs. Hereby, the treatment head, i.e. the radiation unit 10, of the LINAC rotates around the patient 7 (in the RF applicator) requiring approximately one minute per rotation and modulating the dose by switching the leafs on and off every 5°. The multi-leaf collimator 17 consists of 64 leaves with each leaf width 6.25mm in the isocenter covering a field of 40cm. The dose distribution is generated in one slice per rotation adjusting slice thickness between 0.5 to 5cm. Therefore, a volume of 30cm in longitudinal direction can be irradiated in 6 minutes (5cm slices) to 60 minutes (0.5cm slices).

The exact position of the patient 7 relative to the LINAC is controlled using the megavolt computer tomography (MV-CT). The imaging is performed using a detector array of 738 xenon detectors opposite to the central beam of the LINAC. The treatment CT needs 12s per slice, i.e. 1 to 5 minutes for the whole volume. Then, the planning kilovolt computer tomography (kV-CT) dataset is fused with the treatment MV-CT dataset, and a shift vector to correct the position is determined. Note that the planning CT dataset for the patient 7 must be performed in the treatment position, i.e. in the RF applicator. Modern CT scanners for the radiotherapy planning have also gantries of 85cm and enough place to scan a patient positioned in an RF applicator.

After repositioning, the regional hyperthermia is performed in the elliptical RF applicator (modality 1) and the IGRT and IMRT (modality 2) is performed simultaneously with the heat delivery. Typically, a heat-up phase of 20 minutes is required to obtain a plateau for the temperature distribution. Then, a second MV-CT can be performed and further MV-CT during heat delivery in order to characterized the temperature distribution. Therefore, the MV-CT is not only useful for interfractional positioning control with respect to radiotherapy, but also for online control and optimization of the temperature distribution.

Finally, it is important to note that the radio therapy is administered in a conventional fractionation, e.g. 30x2Gy. As a consequence, the thermotherapy can be applied up to 30 times simultaneously. Of course, other combinations of radio therapy and thermotherapy are to consider. Further, a combination with radio chemotherapy is possible.

In the following, the specific characteristics of kV-CT and MV-CT are explained with regard to the invention.

The contrast resolution is =5 HU (Hounsfield units) for 2mm voxel in kV-CT. The HU are directly related to the absorption coefficient HU=1000(µ/µ₀-1). The temperature dependency of density transforms to 0.45HU/°C in muscle (or tumor). The standard fluctuation in one voxel is related to the standard fluctuation σ_{N} in N voxels by σ_{N}=σ · (1+N) ^{-3/4}.

As a consequence, we expect in a conventional CT scanner (140kV) a temperature resolution of only 15°C in a typical voxel of 2mm size. However, in a voxel of 1cm size, the resolution is improved by a factor of 126^{-3/4} , i.e. 37. Therefore, using conventional CT, the temperature resolution is better 0.5°C in 1cm voxels (and can be better 0.1°C in 2cm voxels).

The contrast resolution in MV-CT (3MV X-rays) is described as 15HU (1.5%) in 3cm sized voxels for a non-neglectable dose exposition of 10-12cGy (100-120mSv). This is a temperature resolution of only 30°C. For 6cm objects, we obtain a temperature resolution of 6.5°C, we can differentiate between regions 37-44°C and regions >44°C. This is sufficient to detect hot spots >44°C of some extension (>5cm).

For larger objects of 9cm extension we can even improve the temperature resolution of the mean temperature down to <3°C, i.e. we can differentiate temperature ranges 37-40°C, 40-43°C and above 43°C (for larger regions ≈9cm). This is already sufficient to utilize a code which has been developed to adapt antenna functions of the hyperthermia applicator and to improve (optimize) the temperature distribution according to any prescribed objective function. This algorithm has been originally developed for MR-controlled regional hyperthermia (see Weihrauch et al. 2007 med. Phys.) but can be employed accordingly for a CT-based control.

Then, there are the following options to implement a CT-control for multi-antenna RF-hyperthermia:
Firstly, thermal hot spots can be detected and the temperature distribution can be optimized by using MV-CT at 3MV (e.g. 3°C for 9cm objects).

Another option is an online (real-time) optimization of the heat treatment using the planning kV-CT (140kV) with much better resolution (<0.5°C in 1cm objects).

Finally, a kV-CT can be integrated into the tomotherapy apparatus. Then, the on-board CT can be used for a real-time optimization as mentioned above.

### List of reference numerals:

- 1: Therapeutic device
- 2: Tomotherapy apparatus
- 3: Treatment table
- 4: Rod
- 5: Rod
- 6: Mat
- 7: Patient
- 8: Thermotherapeutic heating device
- 9: Aperture
- 10: Radiation unit
- 11: Radiation detector
- 12: Target
- 13: Electron beam
- 14: Primary collimator
- 15: Flattening filter
- 16: Ion chamber
- 17: Multi-leaf collimator
- 18: Shielding
- 19: Rolls
- 20: Antenna arrangement
- 21: Water bolus
- 22: Radiation therapy apparatus
- 23: Megavolt computer tomograph
- 24: Kilovolt computer tomograph
- 25: Control unit

## Claims

1. Therapeutic device (1) for treatment of a patient (7), particularly for cancer treatment, comprising:
a) a radiation therapy apparatus (2, 22) for applying an ionizing radiation to the patient (7),
b) an integrated thermotherapeutic heating device (8) for inducing a regional hyperthermia in the patient (7),
c) a control unit (25) controlling the operation of the radiation therapy apparatus (2, 22) and the thermotherapeutic device according to a predetermined program which is executed in the control unit (25),
**characterized by**
d) a megavolt computer tomograph (23) adapted to generate body images of the patient (7) for detecting temperature hot-spots caused by the thermotherapeutic heating device (8), wherein the control unit (25) is adapted to perform an image guided control of the thermotherapeutic heating device (8) in such a way that temperature hotspots are avoided or at least resolved, and/or
e) a kilovolt computer tomograph (24) adapted to generate body images of the patient (7) for a high-resolution determination of the temperature distribution within the patient (7), wherein the control unit (25) is adapted to perform an image guided control of the thermotherapeutic heating device (8) in such a way that the actual spatial temperature distribution approximates a desired spatial temperature distribution.

2. Therapeutic device (1) according to claim 1, wherein the radiation therapy apparatus (2, 22) and the thermotherapeutic heating device (8) are adapted to operate simultaneously, so that a regional area of treatment within the patient (7) can be subjected to a radiation therapy and a regional hyperthermia simultaneously.

3. Therapeutic device (1) according to any of the preceding claims, wherein the radiation therapy apparatus (2, 22)
a) is adapted to administer an external beam radiotherapy to the patient (7), and/or
b) is adapted to administer an intensity modulated radiation therapy to the patient (7), and/or
c) is adapted to administer an image guided radiation therapy to the patient (7), and/or
d) comprises a multi-leaf collimator (17) for matching the radiation field to the shape of a tumor, and/or
e) is a tomotherapy apparatus (2) for an image guided delivery of beams of the ionizing radiation helically to the patient (7) from different directions.

4. Therapeutic device (1) according to any of the preceding claims, wherein the thermotherapeutic heating device (8)
a) is adapted to induce the regional hyperthermia by radiating ultrasound, electromagnetic waves, particularly radio frequency waves or microwaves, into the patient (7), and/or
b) comprises an antenna arrangement (20) for radiating the electromagnetic waves into the patient (7), wherein the antenna arrangement (20) is preferably annular or ring-shaped and surrounds the patient (7), or
c) comprises a probe which can be inserted into the patient (7) to an area of treatment, wherein the probe locally heats the patient (7) in the area of treatment.

5. Therapeutic device (1) according to any of the preceding claims, wherein
a) the program provides a first phase during which the thermotherapeutic heating device (8) is switched on and the radiation therapy apparatus (2, 22) is switched off,
b) the program provides a second phase following the first phase, wherein both the thermotherapeutic heating device (8) and the radiation therapy apparatus (2, 22) are switched on during the second phase,
c) the program provides a third phase following the second phase, wherein the thermotherapeutic heating device (8) is switched on and the radiation therapy apparatus (2, 22) is switched off during the third phase.

6. Therapeutic device (1) according to claim 5, wherein
a) the first phase, the second phase and the third phase of the treatment each have a duration in a range between 5 minutes and 30 minutes and more preferably in a range between 15 minutes and 25 minutes, and/or
b) the first phase, the second phase and the third phase have a total accumulated duration in a range between 30 minutes and 90 minutes.

7. Therapeutic device (1) according to any of the preceding claims, wherein the control unit (25) is adapted to control the position of the regional areas of treatment of the radiation therapy apparatus (22) and the thermotherapeutic heating device (8) in such a way that the regional area of treatment of the radio therapy apparatus spatially overlaps with the regional area of treatment of the thermotherapeutic heating device (8).

8. Therapeutic device (1) according to any of the preceding claims, further comprising a body scanner (23, 24) generating images of the body interior of the patient (7).

9. Therapeutic device (1) according to claim 8, wherein the body scanner (23, 24) is
a) a computer tomograph, particularly a kilovolt computer tomograph (24) or a megavolt computer tomograph (23),
b) a magnet resonance tomograph,
c) a positron emission tomograph,
d) an X-ray apparatus,
e) an ultrasonograph,
f) an ultrasound tomograph or
g) a combination of different imaging devices as specified above under a) to f), wherein the images of the different imaging devices are fused.

10. Therapeutic device (1) according to claim 9, wherein
a) the control unit (25) is adapted to control the thermotherapeutic heating device (8) and/or the radiation therapy apparatus (2, 22) based on the images generated by the megavolt computer tomograph (23) and/or the kilovolt computer tomograph (24), and/or
b) the control unit (25) performs an image guided control of the radiation therapy apparatus (2, 22) in such a way that the actual spatial dose distribution approximates a desired spatial dose distribution.

11. Therapeutic device (1) according to claim 10, wherein the thermotherapeutic heating device (8) and/or the radiation therapy apparatus (2, 22) are controlled in real-time and/or during the therapy.

## Patentansprüche

1. Therapeutisches Gerät (1) zur Behandlung eines Patienten (7), insbesondere zur Krebsbehandlung, umfassend:
a) eine Strahlentherapievorrichtung (2, 22) zum Applizieren einer ionisierenden Strahlung auf den Patienten (7),
b) ein integriertes thermotherapeutisches Erwärmungsgerät (8) zum Induzieren einer regionalen Hyperthermie in dem Patienten (7),
c) eine Steuereinheit (25), die den Betrieb der Strahlentherapievorrichtung (2, 22) und des thermotherapeutischen Geräts gemäß einem vorbestimmten Programm steuert, das in der Steuereinheit (25) ausgeführt wird, **gekennzeichnet durch**
d) einen Megavolt-Computertomographen (23), der angepasst ist, um Körperbilder des Patienten (7) zu erzeugen, um Temperatur-Hot-Spots zu erfassen, die **durch** das thermotherapeutische Erwärmungsgerät (8) verursacht werden, wobei die Steuereinheit (25) angepasst ist, um eine bildgeführte Steuerung des thermotherapeutischen Erwärmungsgeräts (8) in der Weise durchzuführen, dass Temperatur-Hot-Spots vermieden oder zumindest beseitigt werden, und/oder
e) einen Kilovolt-Computertomographen (24), der angepasst ist, um Körperbilder des Patienten (7) für eine hochauflösende Bestimmung der Temperaturverteilung innerhalb des Patienten (7) zu erzeugen, wobei die Steuereinheit (25) angepasst ist, um eine bildgeführte Steuerung des thermotherapeutischen Erwärmungsgeräts (8) in der Weise durchzuführen, dass die tatsächliche räumliche Temperaturverteilung eine gewünschte räumliche Temperaturverteilung annähert.

2. Therapeutisches Gerät (1) gemäß Anspruch 1, wobei die Strahlentherapievorrichtung (2, 22) und das thermotherapeutische Erwärmungsgerät (8) angepasst sind, um gleichzeitig zu arbeiten, so dass ein regionaler Behandlungsbereich innerhalb des Patienten (7) gleichzeitig einer Strahlentherapie und einer regionalen Hyperthermie ausgesetzt werden kann.

3. Therapeutisches Gerät (1) gemäß einem der vorangehenden Ansprüche, wobei die Strahlentherapievorrichtung (2, 22)
a) angepasst ist, um eine externe Strahlenradiotherapie auf den Patienten (7) zu applizieren, und/oder
b) angepasst ist, um eine intensitätsmodulierte Strahlentherapie auf den Patienten (7) zu applizieren, und/oder
c) angepasst ist, um eine bildgeführte Strahlentherapie auf den Patienten (7) zu applizieren, und/oder
d) einen Multilamellen-Kollimator (17) aufweist zum Anpassen des Strahlenfeldes an die Form eines Tumors, und/oder
e) eine Thermotherapievorrichtung (2) ist für eine bildgeführte Abgabe von Strahlen der ionisierenden Strahlung in Spiralform auf den Patienten (7) aus verschiedenen Richtungen.

4. Therapeutisches Gerät (1) gemäß einem der vorangehenden Ansprüche, wobei das thermotherapeutische Erwärmungsgerät (8)
a) angepasst ist, um die regionale Hyperthermie zu induzieren durch Abstrahlung von Ultraschall, elektromagnetischen Wellen, insbesondere Hochfrequenzwellen oder Mikrowellen, in den Patienten (7), und/oder
b) eine Antennenanordnung (20) aufweist zum Abstrahlen der radiomagnetischen Wellen in den Patienten (7), wobei die Antennenanordnung (20) vorzugsweise kranzförmig oder ringförmig ist und den Patienten (7) umgibt, oder
c) eine Sonde aufweist, die in dem Patienten (7) bis zu einem Behandlungsbereich eingeführt werden kann, wobei die Sonde den Patienten (7) in dem Behandlungsbereich lokal erwärmt.

5. Therapeutisches Gerät (1) gemäß einem der vorangehenden Ansprüche, wobei
a) das Programm eine erste Phase bereitstellt, innerhalb derer das thermotherapeutische Erwärmungsgerät (8) angeschaltet ist und die Strahlentherapievorrichtung (2, 22) ausgeschaltet ist,
b) das Programm eine zweite Phase bereitstellt im Anschluss an die erste Phase, wobei sowohl das thermotherapeutische Erwärmungsgerät (8) als auch die Strahlentherapievorrichtung (2, 22) während der zweiten Phase angeschaltet sind,
c) das Programm eine dritte Phase bereitstellt im Anschluss an die zweite Phase, wobei während der dritten Phase das thermotherapeutische Erwärmungsgerät (8) angeschaltet und die Strahlentherapievorrichtung (2, 22) ausgeschaltet ist.

6. Therapeutisches Gerät (1) gemäß Anspruch 5, wobei
a) die erste Phase, die zweite Phase und die dritte Phase der Behandlung jeweils eine Dauer haben im Bereich zwischen 5 Minuten und 30 Minuten und vorzugsweise in einem Bereich zwischen 15 Minuten und 25 Minuten und/oder
b) die erste Phase, die zweite Phase und die dritte Phase eine akkumulierte Gesamtdauer im Bereich zwischen 30 Minuten und 90 Minuten haben.

7. Therapeutisches Gerät (1) gemäß einem der vorangehenden Ansprüche, wobei die Steuereinheit (25) angepasst ist, um die Position des regionalen Behandlungsbereichs der Strahlentherapievorrichtung (22) und des thermotherapeutischen Erwärmungsgeräts (8) in einer solchen Weise zu steuern, dass der regionale Behandlungsbereich der Strahlentherapievorrichtung sich räumlich mit dem regionalen Behandlungsbereich des thermotherapeutischen Erwärmungsgeräts (8) überlappt.

8. Therapeutisches Gerät (1) gemäß einem der vorangehenden Ansprüche, weiterhin aufweisend einen Körperscanner (23, 24), der Bilder des Körperinneren des Patienten (7) erzeugt.

9. Therapeutisches Gerät (1) gemäß Anspruch 8, wobei der Körperscanner (23, 24)
a) ein Computertomograph ist, insbesondere ein Kilovolt-Computertomograph (24) oder ein Megavolt-Computertomograph (23),
b) ein Magnetresonanztomograph ist,
c) eine Positronemissionstomograph ist,
d) ein Röntgengerät ist,
e) ein Ultraschallgerät ist,
f) ein Ultraschalltomograph ist, oder
g) eine Kombination von verschiedenen bildgebenden Geräten, wie vorstehend spezifiziert unter a) bis f) ist, wobei die Bilder der verschiedenen bildgebenden Vorrichtungen verschmolzen werden.

10. Therapeutisches Gerät (1) gemäß Anspruch 9, wobei
a) die Steuereinheit (25) angepasst ist, um das thermotherapeutische Erwärmungsgerät (8) und/oder die Strahlentherapievorrichtung (2, 22) zu steuern, basierend auf den Bildern, die von dem Megavolt-Computertomographen (23) und/oder von dem Kilovolt-Computertomographen (24) erzeugt werden, und/oder
b) die Steuereinheit (25) eine bildgeführte Steuerung der Strahlentherapievorrichtung (2, 22) in einer solchen Weise ausführt, dass eine tatsächliche räumliche Dosisverteilung einer gewünschten räumlichen Dosisverteilung annähert.

11. Therapeutisches Gerät (1) gemäß Anspruch 10, wobei das thermotherapeutische Erwärmungsgerät (8) und/oder die Strahlentherapievorrichtung (2, 22) in Echtzeit und/oder während der Therapie gesteuert werden.

## Revendications

1. Dispositif thérapeutique (1) pour le traitement d'un patient (7), particulièrement pour le traitement du cancer, comprenant :
a) un dispositif de thérapie par rayonnement (2, 22) pour appliquer un rayonnement ionisant au patient (7),
b) un dispositif de chauffage thermothérapeutique intégré (8) pour induire une hyperthermie régionale dans le patient (7),
c) une unité de commande (25) commandant le fonctionnement du dispositif de thérapie par rayonnement (2, 22) et du dispositif thermothérapeutique conformément à un programme prédéterminé qui est exécuté dans l'unité de commande (25),
**caractérisé par** :
d) un tomodensitomètre de l'ordre du mégavolt (23) conçu pour générer des images du corps du patient (7) pour détecter des points de haute température provoqués par le dispositif de chauffage thermothérapeutique (8), dans lequel l'unité de commande (25) est conçue pour effectuer une commande guidée par une image du dispositif de chauffage thermothérapeutique (8) de manière à ce que des points de haute température soient évités ou au moins résolus, et/ou
e) un tomodensitomètre de l'ordre du kilovolt (24) conçu pour générer des images du corps du patient (7) pour une détermination à haute résolution de la répartition de la température dans le patient (7), dans lequel l'unité de commande (25) est conçue pour effectuer une commande guidée par une image du dispositif de chauffage thermothérapeutique (8) de manière à ce que la répartition spatiale réelle de la température s'approche d'une répartition spatiale souhaitée de la température.

2. Dispositif thérapeutique (1) selon la revendication 1, dans lequel le dispositif de thérapie par rayonnement (2, 22) et le dispositif de chauffage thermothérapeutique (8) sont conçus pour fonctionner simultanément, de sorte qu'une zone régionale de traitement dans le patient (7) puisse être soumise à une thérapie par rayonnement et à une hyperthermie régionale simultanément.

3. Dispositif thérapeutique (1) selon l'une quelconque des revendications précédentes, dans lequel le dispositif de thérapie par rayonnement (2, 22)
a) est conçu pour administrer une radiothérapie par faisceau externe au patient (7), et/ou
b) est conçu pour administrer une thérapie par rayonnement modulé en intensité au patient (7), et/ou
c) est conçu pour administrer une thérapie par rayonnement guidée par une image au patient (7), et/ou
d) comprend un diaphragme de profondeur (17) pour faire correspondre le champ de rayonnement à la forme d'une tumeur, et/ou
e) est un appareil de tomothérapie (2) pour une distribution guidée par une image de faisceaux du rayonnement ionisant de manière hélicoïdale au patient (7) dans différentes directions.

4. Dispositif thérapeutique (1) selon l'une quelconque des revendications précédentes, dans lequel le dispositif de chauffage thermothérapeutique (8)
a) est conçu pour induire l'hyperthermie régionale en rayonnant des ultrasons, des ondes électromagnétiques, en particulier des ondes radiofréquences ou des ondes hyperfréquences, dans le patient (7), et/ou
b) comprend un agencement d'antenne (20) pour rayonner les ondes électromagnétiques dans le patient (7), dans lequel l'agencement d'antenne (20) est de préférence annulaire ou en forme d'anneau et entoure le patient (7), ou
c) comprend une sonde qui peut être insérée dans le patient (7) dans une zone de traitement, dans lequel la sonde chauffe localement le patient (7) dans la zone de traitement.

5. Dispositif thérapeutique (1) selon l'une quelconque des revendications précédentes, dans lequel
a) le programme exécute une première phase pendant laquelle le dispositif de chauffage thermothérapeutique (8) est activé et le dispositif de thérapie par rayonnement (2, 22) est désactivé,
b) le programme exécute une deuxième phase à la suite de la première phase, pendant laquelle le dispositif de chauffage thermothérapeutique (8) et le dispositif de thérapie par rayonnement (2, 22) sont tous deux activés pendant la deuxième phase,
c) le programme exécute une troisième phase à la suite de la deuxième phase, pendant laquelle le dispositif de chauffage thermothérapeutique (8) est activé et le dispositif de thérapie par rayonnement (2, 22) est désactivé pendant la troisième phase.

6. Dispositif thérapeutique (1) selon la revendication 5, dans lequel
a) la première phase, la deuxième phase et la troisième phase du traitement ont chacune une durée dans une plage entre 5 minutes et 30 minutes et, plus préférablement, dans une plage entre 15 minutes et 25 minutes, et/ou
b) la première phase, la deuxième phase et la troisième phase ont une durée cumulée totale dans une plage entre 30 minutes et 90 minutes.

7. Dispositif thérapeutique (1) selon l'une quelconque des revendications précédentes, dans lequel l'unité de commande (25) est conçue pour commander la position des zones régionales de traitement du dispositif de thérapie par rayonnement (22) et du dispositif de chauffage thermothérapeutique (8) de manière à ce que la zone régionale de traitement du dispositif de radiothérapie recouvre spatialement la zone régionale de traitement du dispositif de chauffage thermothérapeutique (8).

8. Dispositif thérapeutique (1) selon l'une quelconque des revendications précédentes, comprenant en outre un scanner corporel (23, 24) générant des images de l'intérieur du corps du patient (7).

9. Dispositif thérapeutique (1) selon la revendication 8, dans lequel le scanner corporel (23, 24) est
a) un tomodensitomètre, en particulier un tomodensitomètre de l'ordre du kilovolt (24) ou un tomodensitomètre de l'ordre du mégavolt (23),
b) un tomographe à résonance magnétique,
c) un tomographe à positrons,
d) un dispositif à rayons X,
e) un échographe,
f) un tomographe à ultrasons, ou
g) une combinaison de différents dispositifs d'imagerie tels que spécifiés ci-dessus de a) à f), dans laquelle les images des différents dispositifs d'imagerie sont fusionnées.

10. Dispositif thérapeutique (1) selon la revendication 9, dans lequel
a) l'unité de commande (25) est conçue pour commander le dispositif de chauffage thermothérapeutique (8) et/ou le dispositif de thérapie par rayonnement (2, 22) sur la base des images générées par le tomodensitomètre de l'ordre du mégavolt (23) et/ou le tomodensitomètre de l'ordre du kilovolt (24), et/ou
b) l'unité de commande (25) effectue une commande guidée par une image du dispositif de thérapie par rayonnement (2, 22) de manière à ce que la répartition spatiale réelle de dose s'approche d'une répartition spatiale souhaitée de dose.

11. Dispositif thérapeutique (1) selon la revendication 10, dans lequel le dispositif de chauffage thermothérapeutique (8) et/ou le dispositif de thérapie par rayonnement (2, 22) sont commandés en temps réel et/ou pendant la thérapie.
